# EUROPEAN PATENT APPLICATION

(11) **EP 1 961 443 A1**
(43) Date of publication of application: **27.08.2008**
(21) Application number: 08101148.8
(22) Date of filing: 31.01.2008
(51) Int. Cl.: A61M 39/04, A61M 39/22

(54) **Liquid co-injection apparatus**

(30) Priority: 21.02.2007 JP 2007040327
(71) Applicant: Covidien AG, 8212 Neuhausen am Rheinfall (CH)
(72) Inventor: Abe, Kazuhiro, Fukuroi-shi Shizuoka (JP); Funamura, Shigeaki, Fukuroi-shi Shizuoka (JP); Kitani, Ichiro, Fukuroi-shi Shizuoka (JP)
(74) Representative: Körber, Martin Hans

(57) **Abstract**

[PROBLEM TO BE SOLVED] To provide a liquid co-injection apparatus comprising a high pressure resistant rubber plug capable of maintaining the obstructed condition of the slit, even when the hydraulic pressure in the chamber part is increased with the slit of the rubber plug in the obstructed condition.

[SOLUTION] The co-injection apparatus main body 10 of the liquid co-injection apparatus-A comprised of the chamber part 11, the downstream branch-tube 12 extending from the chamber part 11 to different directions respectively, the upstream branch-tube 14, the connection opening 13 and the lid member 20 attached to the connection opening 13. And, the rubber plug 25 is attached to the inside of the lid member 20 to allow a lid member to be obstructed by closing the slit 25a of the rubber plug 25, and to allow the inside of the chamber part 11 to communicate with the inside of the connecting tube by allowing the connecting tube to be inserted into the slit 25a. Moreover, the rib 29a or the like is provided on the outer peripheral surface of the rubber plug 25 to allow the rubber plug 25 to be strongly drawn and pressed onto the inner peripheral surface of the lid member 20, further securing the obstructed condition of the slit 25a.

## Description

### [Technical Field]

The present invention relates to a liquid co-injection apparatus connected to plural infusion tubes or the like used for medical purposes for flowing drug solutions or the like through each of the infusion tubes.

### [Background Art]

Traditionally, plural infusion tubes have been used to supply the desired drug solutions, saline or the like into the patient's body, in such cases, liquid co-injection apparatuses such as medical stopcocks have been used to open-up or shut-off the communication between each of the infusion tubes. Among such foregoing liquid co-injection apparatuses, there exist apparatuses having plural branch-tubes, to which a rubber plug puncturable by injection needles or the like is attached inside the predetermined branch-tubes out of those branch-tube (for example, see Patent Literature 1).
[Patent Literature 1] Unexamined Patent Publication 2004-16437

This liquid co-injection apparatus comprises 2 branch-tubes extending from a main body portion in the horizontal direction and an injection device connecting port formed at the upper part of the main body portion. Also, a seal valve body, in which an inserting part vertically passing through is formed, is provided at this injection device connecting port. Consequently, the inserting part of the seal valve body can be inserted into a twist-lock injection device to allow the twist-lock injection device to communicate with inside a liquid co-injection apparatus main body. Thereby, drug solutions can be injected from the twist-lock injection device to the inside of the liquid co-injection apparatus main body, and furthermore, the drug solutions can be extracted from the liquid co-injection apparatus main body as well. Moreover, while the twist-lock injection device is not inserted into the inserting part of the seal valve body, the seal valve body is obstructed, and the communication between the main body portion and the injection device connecting port is shut-off. In this condition, the drug solutions can be flown from one side to the other side of the 2 branch-tubes

### [Disclosure of the Invention]

In the aforementioned conventional liquid co-injection apparatus however, the seal valve body is formed in an approximately disc-shape, and a portion where the seal valve body is attached in the injection device connecting port, is comprised of the concave part in a shape corresponding to the shape of the seal valve body. Consequently, it is difficult to allow the seal valve body to have a sufficiently large compression force for obstructing the inserting part. As a result, in cases such as when flowing the drug solution from one side to the other side of the 2 branch-tubes with the seal valve body in the obstructed condition, and if the amount of the drug solutions is increased to increase the hydraulic pressure, there may be a possibility that the obstructed condition of the inserting part of the seal valve body can no longer be maintained.

The present invention is made in consideration of the foregoing situations, and the purpose is to provide a liquid co-injection apparatus comprising a high pressure resistant rubber plug capable of maintaining the obstructed condition of the slit, even when the hydraulic pressure in the chamber part is increased with the slit of the rubber plug in the obstructed condition.

The constitutional characteristic of a liquid co-injection apparatus according to the present invention for achieving the aforementioned purpose is that a compression projection part for further securing the obstructed condition of the slit when said connecting tube is not insert into the slit, by further forcing the rubber plug so as to be drawn and pressed onto the inner peripheral surface of the upper part branch-tube, is formed at the predetermined portion of the rubber plug, wherein the liquid co-injection apparatus comprises: a chamber part, and plural branch-tubes including an upper part branch-tube extending from the chamber part to different directions respectively and extending at least upward; and a rubber plug attached to an inner peripheral surface of the upper part branch-tube, for shutting-off the upper part branch-tube by closing a slit passing through the inside as well as for allowing to communicate between inside the chamber part and inside the connecting tube by inserting a connecting tube through said slit.

In the liquid co-injection apparatus of the present invention constituted as described above, when the rubber plug is attached to the inner peripheral surface of the upper part branch-tube, the rubber plug thereof receives a compression force from the inner peripheral surface of the upper part branch-tube to obstruct the slit. And, this rubber plug is provided with a compression projection part for further securing the obstructed condition of the slit by further forcing the rubber plug so as to be drawn and pressed onto the inner peripheral surface of the upper part branch-tube. Consequently, the rubber plug is pressed down by a greater compression force from the inner peripheral surface of the upper part branch-tube so as to be constricted, further securing the obstructed condition of the slit. As a result, even when inside the chamber part is under high-pressure, the obstructed condition of the rubber plug can be maintained.

Moreover, another constitutional characteristic of the liquid co-injection apparatus according to the present invention is that the compression projection part is comprised of a rib formed on the outer peripheral surface of a portion where the slit is formed in the rubber plug. The rib in this case may be one of ribs formed in various shapes such as a rib extending in parallel to the slit in the vertical (up/down) direction, a rib extending along the circumference of the outer peripheral surface of the rubber plug so as to surround the slit, however, preferably a rib extending in parallel to the slit. Moreover, the number of the rib can be one or more, however, preferably plural ribs are formed around the slit spaced one another. This allows the obstructed condition of the slit to be effectively maintained.

Moreover, another constitutional characteristic of the liquid co-injection apparatus according to the present invention is that the rubber plug main body portion is comprised of a slit forming part where the slit is formed and a dome-shaped part expanding from the lower end peripheral part to below the outer peripheral side of the slit forming part; and in addition, a bulging-out part protruding downward at the lower end peripheral part and the adjacent portion of the slit in the under surface of the dome-shaped part, is formed; and the compression projection part is comprised of the bulging-out part.

This allows, when the rubber plug receives a pressure from the chamber part side, the compression projection part to be pressed upward by the pressure so as to strongly compress the slit. Consequently, the sealing effect of the rubber plug is further enhanced. In this case, it is preferred that the inner peripheral surface of the upper part branch-tube is formed in a tapered surface having a diameter becoming gradually reduced from the lower part side to the upper part side, and at the same time, the outer peripheral surface of the slit forming part in the rubber plug is formed in a tapered surface having a diameter becoming gradually reduced from the lower part side to the upper part side. This allows, when the rubber plug receives a pressure from the chamber part side, the slit forming part of the rubber plug to be pressed so as to be pushed into the inner peripheral surface of the upper part branch-tube so as to strongly compress the slit. Moreover, the transverse section shapes of the upper part branch-tube and of the rubber plug in this case may be formed in various shapes including a round, egg, triangular and quadrangular shapes.

### [Best Mode for Carrying Out the Invention.]

### (The First Embodiment)

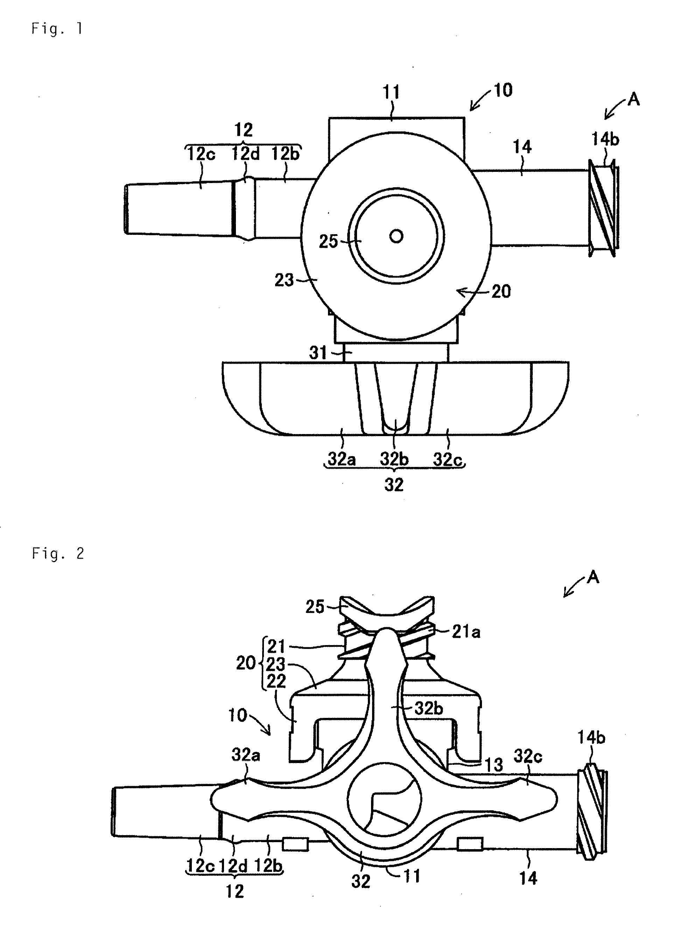

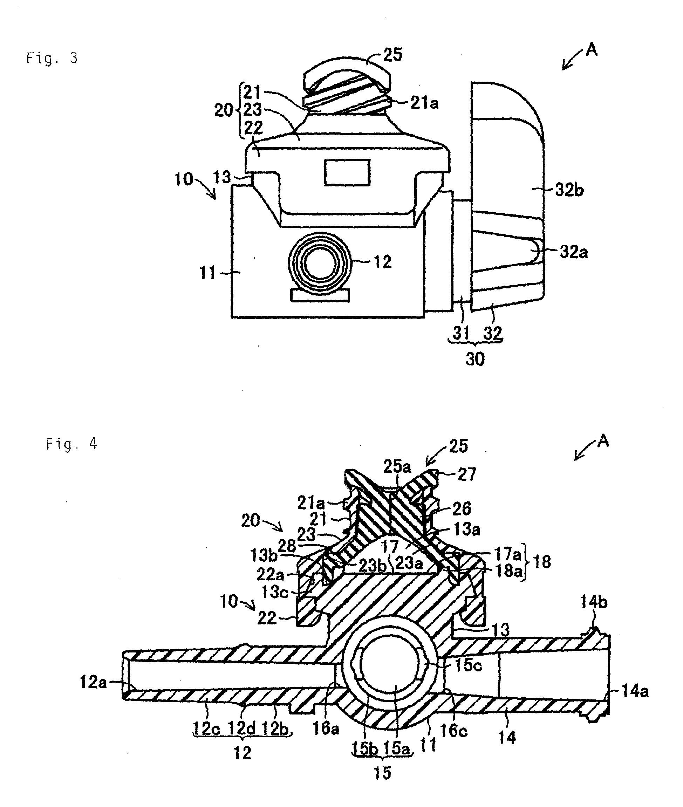

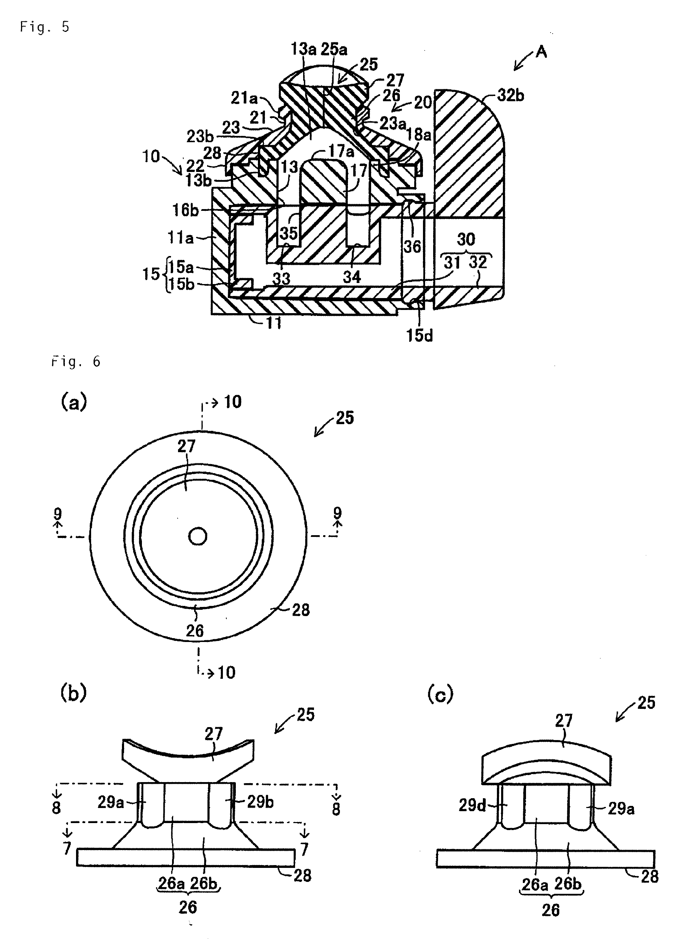

Hereinafter, a liquid co-injection apparatus according to the first embodiment of the present invention will be descried in detail 1 with reference to drawings. Fig. 1 or Fig. 3 shows a liquid co-injection apparatus-A according to the same embodiment, wherein this liquid co-injection apparatus-A is comprised of a co-injection apparatus main body 10, a rubber plug 25 attached to the upper part side of the co-injection apparatus main body 10 and a valve body 30 attached in the co-injection apparatus main body 10 (see Fig. 5). And, the co-injection apparatus main body 10 is comprised of a cylindrical chamber part 11 having a short axial length, a downstream branch-tube 12 connected in sequence to the outer peripheral surface of the chamber part 11 at a 90-degree angle, a connection opening 13, a upstream branch-tube 14 and 3 branch-tubes consisting of a lid member 20 attached to the connection opening 13. Incidentally, the upper part branch-tube of the present invention is comprised of the connection opening 13 and the lid member 20.

The chamber part 11 is formed in an approximately cylindrical shape obstructed at the rear section and is placed with the axial direction in the cross direction (right and left directions in Fig. 3 and Fig. 5). And, the engaging part 15 having a cross-section shape of an approximately sideway U-shape is fixed on an inner surface of a rear wall part 11a of the chamber part 11. This engaging part 15 is comprised of a disc part 15a along an inner surface centre of the rear wall part 11a, and a ring part 15b having a short axial length extending from the outer peripheral part of the disc part 15a to the front, spaced from the inner peripheral surface of the chamber part 11. And, as shown in Fig. 4, a pair of locking projections 15c are formed at both right and left sides in the outer peripheral surface of the ring part 15b. Moreover, an engaging concave part 15d consisting of a narrow groove is formed along the circumference at a front side position in the inner peripheral surface of the chamber part 11.

And, communication holes 16a, 16b and 16c are formed respectively at portions where the downstream branch-tube 12, the connection opening 13 and the upstream branch-tube 14 are connected in the perimeter wall part of the chamber part 11. The communication hole 16b out of these communication holes 16a, 16b and 16c is formed with the centre part displaced slightly to the front side than the centre part of the other communication holes 16a, 16. And, through the use of the communication hole 16a, inside the chamber part 11 and the flow passage 12a formed inside of the downstream branch-tube 12 thereof are communicated, and through the use of the communication hole 16b, inside the chamber part 11 and the flow passage 13a formed inside the connection opening 13 are communicated.

Moreover, through the use of the communication hole 16c, inside the chamber part 11 and the flow passage 14a formed inside the upstream branch-tube 14 are communicated. The downstream branch-tube 12 is integrally formed with the chamber part 11, and is comprised of a base part 12b of the chamber part 11 side and a male luer part 12c of the tip side formed thinner than the base part 12b. And, a protrusion part 12d is formed along the circumference at a boundary part between the base part 12b and the male luer part 12c in the outer peripheral surface of the downstream branch-tube 12.

The diameter of the connection opening 13 constituting the lower part side portion of the upper part branch-tube is larger than those of the downstream branch-tube 12 and the upstream branch-tube 14, and the axial length is formed in a short approximately ring shape. And, an engaging groove 13b is formed at an approximately centre of the width direction in the upper end peripheral part of the connection opening 13 along the circumference, and a pair of engagement projections 13c are formed at both right and left sides in the outer peripheral surface portion respectively. Moreover, a weir part 17 for partitioning the flow passage 13a into a front part and a rear part is formed in spanned condition at both right and left sides of the inner peripheral surface of the connection opening 13, and a pair of rubber plug holding parts 18 are formed at a portion spanning from the inner side portion of the engaging groove 13b in the upper end peripheral part of the connection opening 13 to the upper end face of the weir part 17. This pair of rubber plug holding parts 18 constitute a wall part of the inner peripheral surface side of the engaging groove 13b, and are comprised of a pair (right and left) of side wall portions 18a protruded more upside than an upper surface 17a of the weir part 17, and both right and left sides portions of the top surface 17a of the weir part 17.

Moreover, the lid member 20 for fixing the lower end part of the rubber plug 25 together with the connection opening 13 is attached at the engaging projection 13c of the connection opening 13. An upper part 21 of the lid member 20 is formed in an approximately cylindrical shape having a diameter slightly larger than those of the downstream branch-tube 12 and of the upstream branch-tube 14, having a short axial length. Moreover, a lower part 22 of the lid member 20 is formed in an approximately cylindrical shape having a diameter larger than that of the connection opening 13, having a short axial length, and furthermore having an ellipse shape having a length longer anteroposteriorly in the plane view. And, a sloping part 23 becoming gradually thinner from the lower part 22 side toward the upper part 21 side is formed in between the upper part 21 and the lower part 22.

Moreover, the lower end part of both right and left sides portions of the lower part 22 extends downward lower than the lower end part of the front and rear both sides portion, and a pair of engaging concave parts 22a engageable with the engaging projection 13c of the connection opening 13 is formed on the inner surface of the portion extending downward. Moreover, the upper end opening edge part of the upper part 21, as shown in Fig. 2, is formed in a circular arc shape having a centre side depressed downward when viewed from the front, and as shown in Fig. 3, is formed in a circular arc shape having the centre side protruded upward when viewed from the side. And, a thread 21a is formed on the outer peripheral surface of the upper part 21.

Moreover, a tapered surface 23a becoming gradually thinner from the lower part 22 side toward the upper part 21 side is formed on the inner surface of the sloping part 23, and an engaging stepped part 23b is formed at the lower part side of the tapered surface 23a. This lid member 20 is attached removably to the connection opening 13 by engaging an engaging concave part 22a of the lower part 22 to the engaging projection 13c. Moreover, the lower part side portion of the rubber plug 25 is fixed by way of the engagement between this lid member 20 and the connection opening 13.

The rubber plug 25 is made from an elastic member such as natural rubber, synthetic rubber or elastomer and, as shown in Fig. 6, is comprised of a rubber plug main body 26, an upper fixed piece 27 formed at the upper end of the rubber plug main body 26, and a ring shaped lower fixed piece 28 formed at the outer peripheral side of the lower end peripheral part of the rubber plug main body 26. Moreover, the upper part side of the rubber plug main body 26 is comprised of a slit forming part 26a formed in a thick disc-shape and having a slit 25a formed inside thereof, and the lower part side of the rubber plug main body 26 is comprised of an approximately dome-shaped part 26b. And, the rubber plug 25 is attached to the lid member 20 with the rubber plug main body 26 positioned inside the upper part 21, and the dome-shaped part 26b positioned inside the sloping part 23.

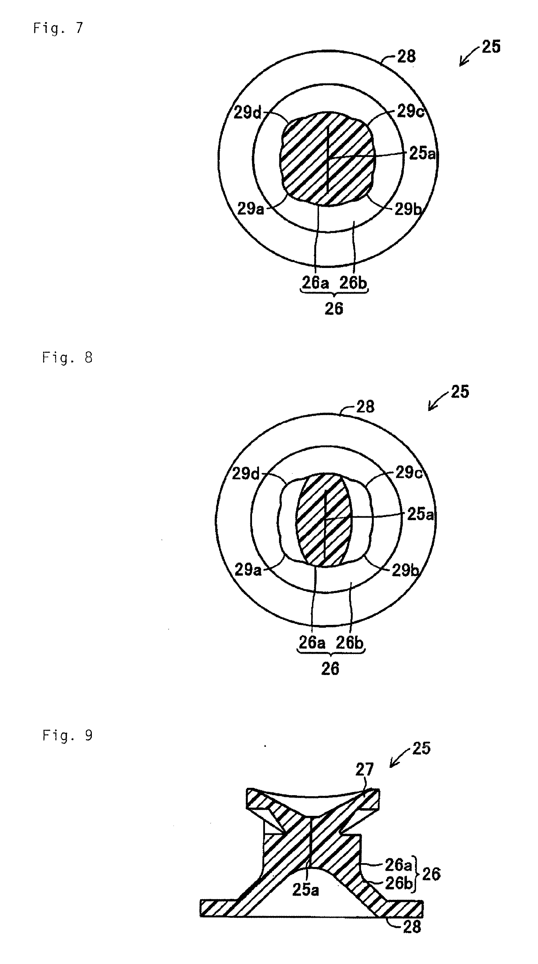

Moreover, 4 ribs 29a, 29b, 29c and 29d (see Fig. 7 and Fig. 8) are formed circumferentially on the outer peripheral surface of the slit forming part 26a keeping a certain distance one another as a compression projection part of the present invention extending in the vertical direction. These ribs 29a, 29b, 29c and 29d having cross-sections formed in a circular arc shape, slightly protrude out of the outer peripheral surface of the slit forming part 26a. Moreover, these ribs 29a, 29b, 29c and 29d are formed spanning from the upper end part of the slit forming part 26a to the top surface of the dome-shaped part 26b.

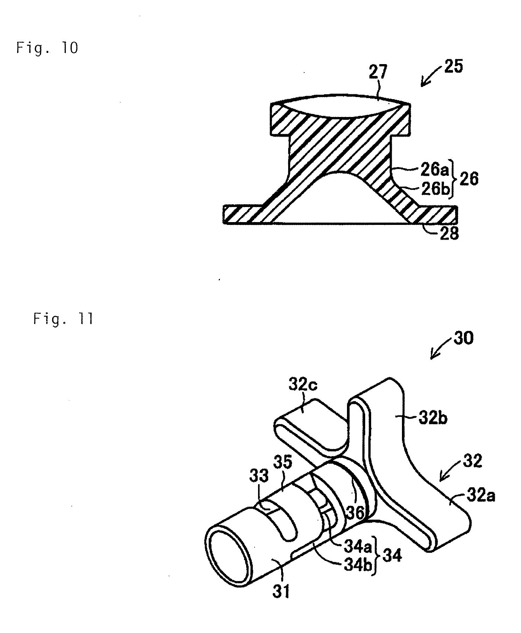

The upper fixed piece 27 is formed in a shape following along the shape of the upper end opening edge part of the lid member 20, and is comprised of an approximately disc shaped body having a rounded surface curved toward an oblique upside orientation of right and left respectively from the center portion in right and left direction (right and left direction in Fig. 6(b)) in the top surface of the slit forming part 26a. Consequently, as shown in Fig. 9, a notch-like gap is formed between the under surface of both right and left sides portions of the upper fixed piece 27 and the both right and left sides portions of the upper surface of the slit forming part 26a. Moreover, as shown in Fig. 10, the boundary portion with the slit forming part 26a in both front and rear sides portions (both right and left sides in Fig. 10) of the upper fixed piece 27 is formed in a larger-diameter than that of a boundary part between the slit forming part 26a and the dome-shaped part 26b. And, the rubber plug 25 constituted as the foregoing is attached to the inside of the lid member 20 in the condition in which the plug is prevented from disengaging from the upper part 21 at a boundary portion between the slit forming part 26a and the upper fixed piece 27.

Moreover, the lower fixed piece 28 of the rubber plug 25, as shown in Fig. 4 and Fig. 5, is fixed by the connection opening 13 and the lid member 20 by making the engaging concave part 22a of the lid member 20 engaged to the engaging projection 13c of the connection opening 13 in the condition in which the peripheral part thereof is inserted into the engaging groove 13b of the connection opening 13. At this time, the top surface of the lower fixed piece 28 is bent along the engaging stepped part 23b, and the under surface of the lower fixed piece 28 is bent along the sidewall portion 18a. Thus, the lower fixed piece 28 is interposed and bent in between the connection opening 13 and the lid member 20, thereby the lower fixed piece 28 is more solidly secured.

More specifically, the rubber plug 25 is prevented from entering into the inside of the lid member 20 by the larger-diameter portion of the boundary between the slit forming part 26a and the upper fixed piece 27, and the lower fixed piece 28 inserted into the engaging groove 13b is fixed by the engagement between the connection opening 13 and the lid member 20, and therefore is prevented from escaping through the upper end opening of the lid member 20 to the outside. Thereby, the rubber plug main body 26, when strongly pressed downward from the upside, can move from the upper part 21 of the lid member 20 downward while being deformed, and when pressed upward from the downside, is pushed against the inner peripheral surface of the upper part 21. Moreover, when the rubber plug main body 26 moves downward (elongation) while being deformed, the upper fixed piece 27 elongates, and the lower fixed piece 28 elongates or deforms.

Moreover, the slit 25a passing through the slit forming part 26a and the upper fixed piece 27 vertically can facilitate the communicate between the inside and the outer side of the connection opening 13 to form a part of the flow passage 13a of the connection opening 13. This slit 25a is maintained in the obstructed condition by a compression force that the rubber plug main body 26 receives from the inner peripheral surface of the lid member 20 upon when the rubber plug 25 is attached to the lid member 20. At that time, the compression force that the rubber plug main body 26 receives from the inner peripheral surface of the lid member 20 becomes greater by the ribs 29a, 29b, 29c and 29d, further securing the obstructed condition of the slit 25a.

And, this slit 25a, into which, for example, the male luer part of the syringe can be inserted to form a flow passage that communicates between the syringe and the flow passage 13a in the male luer part. In this case, the male luer part and the peripheral surface of the slit 25a are closely attached one another by the elasticity of the rubber plug 25. In addition, a portion corresponding to a pair of rubber plug holding parts 18 in the peripheral part of the under surface of the rubber plug main body 26 are pressed against the rubber plug holding part 18 and deformed respectively so as to ensure no gap in which air can be accumulated exists at the tip part side of the male luer part.

Moreover, when the male luer part is not inserted in the slit 25a, the under surface of the dome-shaped part 26b becomes a smooth dome-shaped curved surface so that air cannot be accumulated easily when a liquid such as a drug solution flows under surface side of the dome-shaped part 26b. In addition, even when a large pressure is applied onto the rubber plug main body 26 upward from the downside due to a liquid flowing under surface side of the rubber plug main body 26, the obstructed condition of the slit 25a is maintained more securely by the elasticity of the rubber plug 25 and/or the compression force that the rubber plug 25 receives from the inner peripheral surface of the lid member 20 through the ribs 29a, 29b, 29c and 29d.

The upstream branch-tube 14 is integrally formed with the chamber part 11, and the communication hole 16c side portion of the flow passage 14a formed inside thereof is formed in a tapered shape having a diameter becoming smaller toward the communication hole 16c and the diameter becoming smaller away from the communication hole 16c. Moreover, the upstream portion (the right side portion in Fig. 4) of the flow passage 14a is formed in a tapered shape having a diameter gradually larger toward the openings of the upstream branch-tube 14. The opening side portion of this upstream branch-tube 14 forms a female luer part for connecting the male luer part connected to the other tube or the like. And, the connecting thread part 14b is formed at the outer peripheral surface of the opening of the upstream branch-tube 14.

The valve body 30 is comprised of an approximately cylindrically shaped valve main body 31 and an operating part 32 connected to the front end part of the valve main body 31. And, the valve main body 31 is installed in the chamber part 11 in the condition in which the tip part is inserted in between the inner peripheral surface of the chamber part 11 and the ring part 15b of the engaging part 15, and rotates in the axial direction of the chamber part 11 by manipulating the operating part 32. Moreover, plural locking concave parts (not shown) engageable to a pair of the locking projection 15c formed at the outer peripheral surface of the ring part 15b are formed at the tip part in the inner peripheral surface of the valve main body 31 spaced circumferentially, and predetermined the locking concave part out of these the locking concave part can be engaged to the locking projection 15c to rest the valve body 30 at 3 locations preliminarily setup.

Moreover, an engaging protruded ridge 36 engaging with the engaging concave part 15d of the chamber part 11 is formed at the operating part 32 side in the outer peripheral surface of the valve main body 31 along the circumference, and the valve body 30 is prevented from disengaging from the chamber part 11 by the engagement of this engaging protruded ridge 36 with the engaging concave part 15d. And, as shown in Fig. 11, on the outer peripheral surface of the valve main body 31 2 grooves 33,34 are formed and positioned axially side by side.

The groove 33 is comprised of a notched groove formed over an approximately half-perimeter along the circumference at a portion slightly back side (left side in Fig. 11) from the axial center in the outer peripheral surface of the valve main body 31, Moreover, the groove 34 is comprised of an approximately L-shaped notched grooves consisting of a circumferential groove 34a formed along the outer peripheral surface of the valve main body 31 in parallel to the groove 33 at a portion slightly to front side from the axial center in the outer peripheral surface of the valve main body 31, and an axial groove 34b bent from one side of end parts of the circumferential groove 34a and extending toward the back side in the axial direction.

And, the axial groove 34b is provided at a position spaced from one side of end parts of the groove 33, and the other side of end parts of the circumferential groove 34a is positioned at one side along the circumferential direction than the other side of end parts of the groove 33 (near side in Fig. 11). Moreover, both lengths of the groove 33 and the groove 34 along the circumferential direction of the valve main body 31 are determined equally with the approximately half-perimeter of the circumference, and the distance between the groove 33 and the circumferential groove 34a is nearly the same as the total of the width of the groove 33 and the width of the circumferential groove 34a. And, a partitioning wall part 35 is formed between the groove 33 and the circumferential groove 34a along the outer peripheral surface of the valve main body 31.

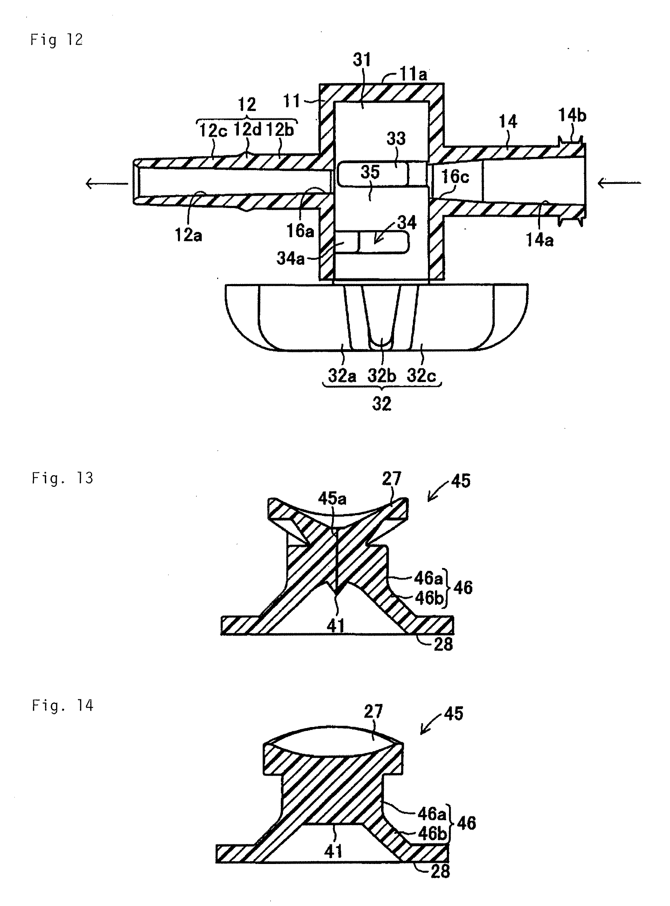

Moreover, the valve main body 31 at the time when installed in the chamber part 11, as shown in Fig. 12, a portion where the groove 33 in the outer peripheral surface and the axial groove 34b of the groove 34 are formed is positioned to the positions of the communication holes 16a, 16c, and a portion where the circumferential groove 34a is formed is opposed to the front side position in the inner peripheral surface of the chamber part 11. And, the outer peripheral surface of the partitioning wall part 35 is opposed to the communication hole 16b at a portion to slightly front side than the centre in the inner peripheral surface of the chamber part 11.

Consequently, when the valve main body 31 is positioned in such a manner that the partitioning wall part 35 faces upward, the groove 33 is opposed to the communication hole 16c and inside the chamber part 11 and the upstream branch-tube 14 are communicated through the use of the groove 33. Moreover, the backside of the axial groove 34b in the groove 34 is opposed to the communication hole 16a and inside the chamber part 11 and the downstream branch-tube 12 are communicated. In this case, the weir part 17 is position at the upside of the partitioning wall part 35, and the outer peripheral surface of the partitioning wall part 35 and the under surface of the weir part 17 come in contact one another in a closely attached condition.

And, because that the open space part constituting the flow passage 13a is positioned at the upside of the weir part 17, the groove 33 and the groove 34 are communicated through the use of the flow passage 13a. Hence, in this condition, drug solutions or the like can be flown from the upstream branch-tube 14 to the downstream branch-tube 12 through the use of the chamber part 11 and the connection opening 13. In this case, drug solutions flowing from the upstream branch-tube 14 into the inside of the groove 33, flow over the weir part 17 and continue to flow into the groove 34. Consequently, the drug solutions or the like pass through inside the flow passage 13a of the upside of the chamber part 11, thereby, accumulations of air or the like in the chamber part 11 and/or in the flow passage 13a can be suppressed.

From the condition thereof, the valve body 30 is rotated to one side so that the groove 33 is opposed to the communication hole 16a, and then the outer peripheral surface of the valve main body 31 is made to oppose to the communication hole 16c, thereby the communication between inside the chamber part 11 and the downstream branch-tube 12 is opened-up and the communication between inside chamber part 11 and the upstream branch-tube 14 is shut-off. Moreover, the valve body 30 is rotated to the other side so that the groove 33 can be kept opposing to the communication hole 16c, and then the outer peripheral surface of the valve main body 31 is made to oppose to the communication hole 16a, the communication between inside the chamber part 11 and the downstream branch-tube 12 is shut-off, and the communication between inside the chamber part 11 and the upstream branch-tube 14 is opened-up.

Thus, by performing a rotating operation of the valve body 30, both the downstream branch-tube 12 and the upstream branch-tube 14 can be made to communicate with inside the chamber part 11 or only one side can be made to communicate with inside the chamber part 11. Incidentally, the operating part 32 comprises 3 operating pieces 32a, 32b and 32c, and these operating pieces 32a, 32b and 32c are formed at a 90-degree angle so as to correspond to the downstream branch-tube 12, the connection opening 13 (the lid member 20) and the upstream branch-tube 14 respectively. And, when the operating piece 32b is positioned to the position of the lid member 20, all of the flow passages 12a, 13a and 14a are communicated, and when the operating piece 32b is positioned to the position of the downstream branch-tube 12, the flow passages 12a, 13a are communicated, and when the operating piece 32b is positioned to the position of the upstream branch-tube 14, the flow passages 13a, 14a are communicated. Moreover, at that time, a locking concave part is engaged to the locking projection 15c and the operating part 32 remains at that position unless otherwise an external force is applied.

In this constitution, in the obstructed condition of the rubber plug 25, in the event that predetermined drug solutions or the likes are injected from the upstream branch-tube 14 to the downstream branch-tube 12, and the drug solutions thereof are supplied into a patient (not shown) body, firstly a rear end part of an infusion tube (not shown) having an indwelling needle for puncturing and indwelling is connected to the downstream branch-tube 12. Then, the male luer part provided at the tip part of the infusion tube extending from a container or the like containing the drug solution to be supplied to the patient is connected to the upstream branch-tube 14. Then, after the drug solution is passed through inside the infusion line including the chamber part 11 to release all the air inside the infusion line, the indwelling needle is punctured into the patient's body and indwelled within, and then the drug solutions in the container or the like is pumped toward the patient, thereby the supply of drug solution into the patient is carried out.

In this case, the drug solution flows from the upstream branch-tube 14 and passes through inside the chamber part 11, and then flows toward the downstream branch-tube 12 side. At that time, the slit 25a of the rubber plug main body 26 is obstructed to prevent the drug solution from leaking out of the rubber plug 25. Moreover, even when the amount of the drug solution is increased causing the hydraulic pressure inside the chamber part 11 to increase, the rubber plug main body 26 can withstand the high pressure by providing the rib 29a or the like, thereby the obstructed condition of the slit 25a can be securely maintained, allowing the drug solution to passing through inside the chamber part 11 in an appropriate condition. Moreover, in the event that 2 kinds of the drug solutions are supplied into a patient (not shown) body, from the aforementioned condition, in the condition in which the other drug solutions is aspirated inside the drug solution containing part of a syringe (not shown), the male luer part of the syringe is passed completely through the slit 25a of the rubber plug 25. At this time, the male luer part presses the inner peripheral surface of the rubber plug main body 26 against the lid member 20 to widen the slit 25a while making the rubber plug main body 26 deformed.

And, the rubber plug main body 26 and the upper fixed piece 27 extend (elongate) downward while deforming. Thereby, the rubber plug main body 26 is pressed against the lid member 20 by a pressing force of the male luer part, extending downward while being closely attached to the inner peripheral surface of the lid member 20 and the outer peripheral surface of the male luer part. And, when the male luer part is inserted into the slit 25a to allow the drug solution containing part to communicate with the flow passage 13a, the lower end part of the rubber plug main body 26 is pressed against the rubber plug holding part 18, no gap is created at the tip side portion of the male luer part where air tends to accumulate.

Then, after the drug solution is passed through inside the infusion line including the chamber part 11 to release all the air inside the infusion line, the indwelling needle is punctured into the patient's body and indwelled within, and then the drug solutions in the container or the like is pumped toward the patient, thereby the supply of drug solution into the patient is carried out. Moreover, the drug solution inside the drug solution containing part of the syringe is also, arbitrarily inject into the of inside of the chamber part 11 through the flow passage 13a. And, when the supply of the drug solution from the syringe is completed and the male luer part is pulled out from the slit 25a, the rubber plug main body 26 is released from the pressing force exerted by the male luer part, retuning to the original condition by its own restoring force. Moreover, according to this liquid co-injection apparatus-A, the inside of the lid member 20 is obstructed by the rubber plug 25, thereby possible propagation of bacteria due to air entering inside the chamber part 11 can also be suppressed.

Thus, with the liquid co-injection apparatus-A according to the Embodiment, the obstructed condition of the rubber plug 25 attached to the lid member 20 is maintained by the compression force received from the inner peripheral surface of the lid member 20, and in addition, the compression force that the rubber plug main body 26 received from the inner peripheral surface of the lid member 20 is increased by the ribs 29a, 29b, 29c and 29d formed on the outer peripheral surface of the slit forming part 26a, further securing the obstruction of the slit 25a. As a result, even when inside the chamber part is under high-pressure, the obstructed condition of the rubber plug can be maintained. Moreover, the rubber plug 25 is made from a deformable elastic member; connecting tubes such as the male luer part can be easily inserted into the slit 25a or the like.

### (The Second Embodiment)

Fig. 13 and Fig. 14 show a substantial part of the rubber plug 45 comprised in a liquid co-injection apparatus according to the second Embodiment of the present invention. The outer shape of this rubber plug 45 is in the same shape as the outer shape of the rubber plug 25 (Fig. 6) comprised in the aforementioned liquid co-injection apparatus-A. And, in this rubber plug 45, the bulging-out part 41 protruding downward is formed on the under surface of the rubber plug main body 46 comprised of the slit forming part 46a and the dome-shaped part 46b. This bulging-out part 41 is symmetrically formed along both sides of the lower end edge part of the slit 45a so that the lower end edge part of the slit 45a is positioned at the lowest part.

And, one side of the cross-section shape shown in Fig. 13 in the bulging-out part 41 is a triangular shape with the lower end edge part of the slit 45a positioned at the lowest part interposing the slit 45a, and the other cross-section shape shown in Fig. 14 (the section perpendicularly intersecting with the section in Fig. 13) is an approximately trapezoid shape of a wide width. The constitutions of other portions in this rubber plug 45 and the liquid co-injection apparatus comprising the rubber plug 45 are the same as those of the aforementioned rubber plug 25 and the liquid co-injection apparatus-A. Thus, the identical notations are assigned to the identical parts and the detailed descriptions are omitted. Incidentally, in this rubber plug 45, the compression projection part is comprised of 4 ribs (not shown in Figures) and the bulging-out part 41.

According to this liquid co-injection apparatus, the obstruction of the slit 45a can be further secured by 4 ribs, and in addition, the sealing effect of the rubber plug 45 can be further improved by the compression effect of the rubber plug main body 46 by the use of the bulging-out part 41. More specifically, according to this rubber plug 45, when the rubber plug 45 receives hydraulic pressure from the chamber part 11 side, the bulging-out part 41 is pressed upward by the hydraulic so as to strongly compress the slit 45a. Thereby, the sealing effect of the rubber plug 45 is further enhanced. Other functions and effects of the liquid co-injection apparatus comprising this rubber plug 45 are same as those of the aforementioned liquid co-injection apparatus-A.

### (Third Embodiment)

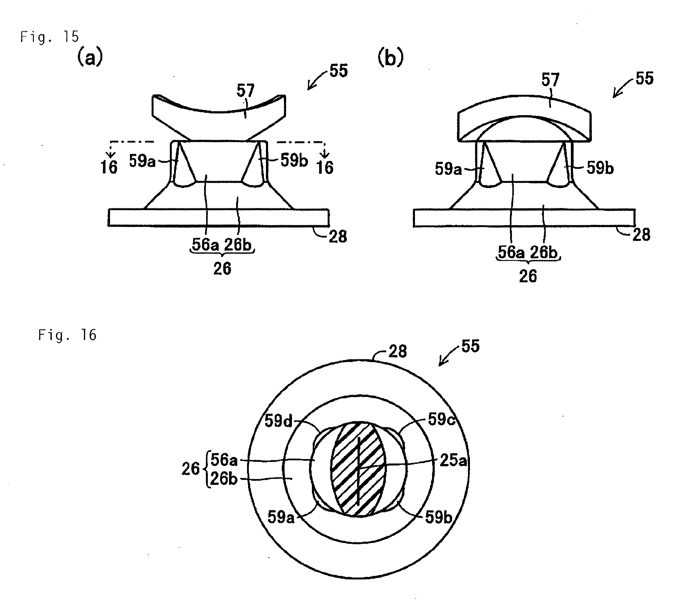

Fig. 15 shows a rubber plug 55 comprised in a liquid co-injection apparatus according to the third embodiment of the present invention. In this rubber plug 55, 4 ribs 59a, 59b, 59c, 59d (see Fig. 16) formed on the outer peripheral surface of the slit forming part 56a becoming gradually thinner from the lower part side to the upper part side respectively. More specifically, the cross-section shapes of the lower end parts of the ribs 59a, 59b, 59c, 59d are the same as the cross-section shapes of the ribs 29a, 29b, 29c and 29d of the rubber plug 25 of the aforementioned first embodiment, and the upper end part of the ribs 59a, 59b, 59c, 59d are points with no area.

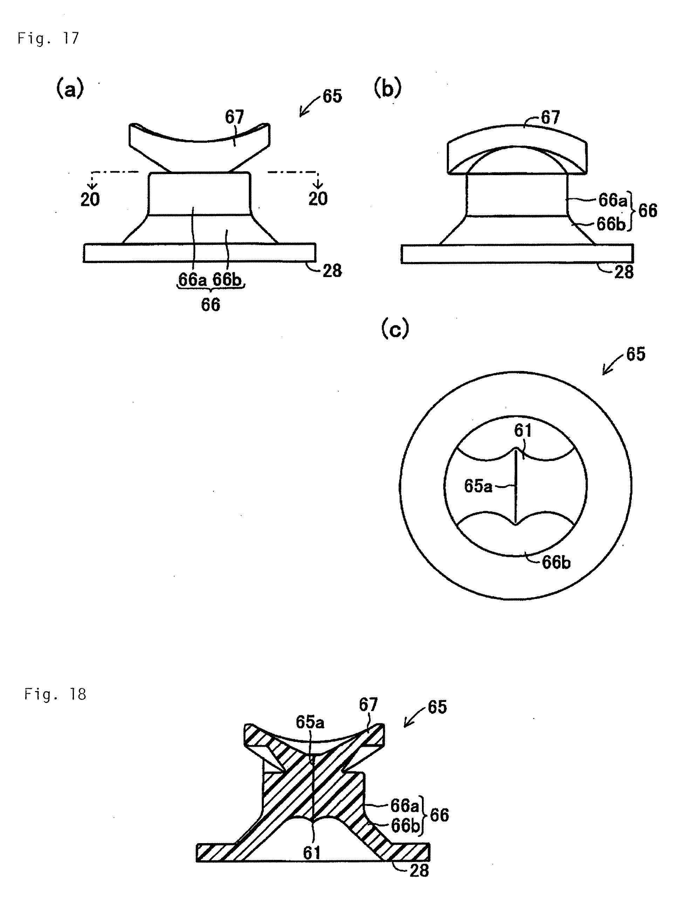

Hence, when viewing the downside portion from the upside and from a boundary part between a slit forming part 56a and an upper fixed piece 57 in the rubber plug 55, is as shown in Fig. 17. The constitutions of other portions in this rubber plug 55 and the liquid co-injection apparatus comprising the rubber plug 55 are the same as those of the aforementioned rubber plug 25 and the liquid co-injection apparatus-A. Thus, the identical notations are assigned to the identical parts and the detailed descriptions are omitted.

According to this rubber plug 55, since the ribs 59a, 59b, 59c, 59d are becoming thinner from the lower part side toward the upper part side, when the rubber plug 55 receives hydraulic pressure from the chamber part 11 side, the slit forming part 56a is pressed upward by the hydraulic pressure, making it easy to enter into the upper part side in the lid member 20. Thereby, a stronger compression force is applied onto the slit 25a, further enhancing the sealing effect of the rubber plug 55. Other functions and effects of the liquid co-injection apparatus comprising this rubber plug 55 are same as those of the aforementioned liquid co-injection apparatus-A. Moreover, as a modified example of this rubber plug 55, the rubber plug 55 may be provided with the bulging-out part 41 comprised in the rubber plug 45 of the aforementioned second Embodiment. This allows to further improve the sealing effect of the rubber plug 55.

### (The Fourth Embodiment)

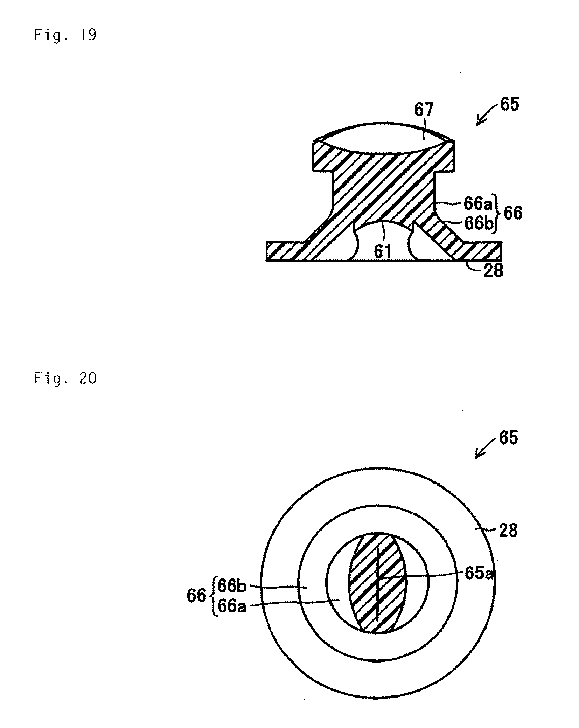

Fig. 17 or Fig. 19 shows a rubber plug 65 comprised in the liquid co-injection apparatus according to the fourth embodiment of the present invention. In this rubber plug 65, the bulging-out part 61 protruding downward is formed on the under surface of the rubber plug main body 66 comprised of a slit forming part 66a and a dome-shaped part 66b. This bulging-out part 61 is symmetrically formed along both sides of the lower end edge part of the slit 65a, in such a manner that the lower end edge part of the slit 65a is positioned at the lowest part. And, the lower end edge part of one side of the cross-section shape shown in Fig. 19 in the rubber plug 65 extending from both sides of the lower end edge part of the slit 65a upward into different directions respectively, curving so as to describe an arc and then extending downward, is formed in a smooth circular arc shape.

Moreover, the lower end edge part of the other cross-section shape shown in Fig. 20 (the cross-section perpendicularly intersected with the cross-section in Fig. 19) in the rubber plug 65 is formed in a smooth circular arc shape with the centre portion concaved. And, small steps is formed respectively between both end parts of the bulging-out part 61 and the under surface of the dome-shaped part 66b. Consequently, when viewing the rubber plug 65 from the bottom surface side, the bulging-out part 61, as shown in Fig. 17(c), appears to be a pair of axes placed with the blade-edges facing outward respectively, interposing the slit 65a. Moreover, the ribs are not provided at the slit forming part 66a of this rubber plug 65. Hence, when viewing the downside portion from the upside from the boundary part between the slit forming part 66a and the upper fixed piece 67 in the rubber plug 65, it appears as shown in Fig. 20.

The constitutions of other portions in this rubber plug 65 and the liquid co-injection apparatus comprising the rubber plug 65 are the same as those of the aforementioned rubber plug 25 and the liquid co-injection apparatus-A. Thus, the identical notations are assigned to the identical parts and the detailed descriptions are omitted. Incidentally, in this rubber plug 65, the compression projection part is comprised of the bulging-out part 61. With the liquid co-injection apparatus comprising this rubber plug 65, functions and effects similar to those of the liquid co-injection apparatus according to each of the aforementioned embodiments may also be obtained.

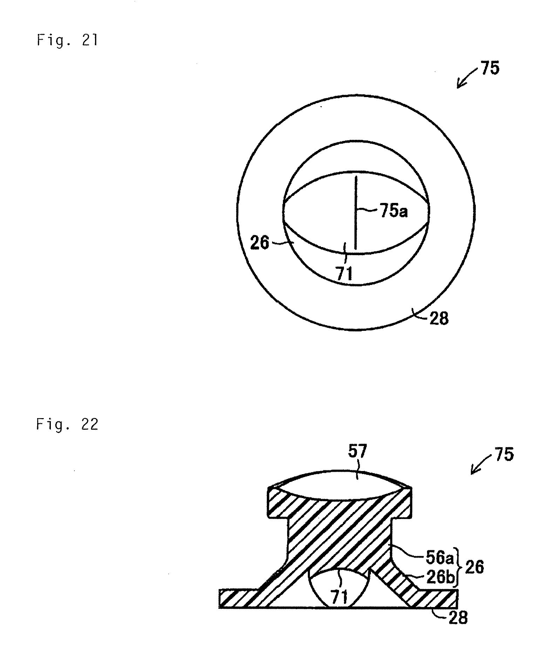

Moreover, as a modified example of the rubber plug 65 comprised in the liquid co-injection apparatus according to the fourth embodiment, a rubber plug 75 having a bottom surface shown in Fig. 21, and having a cross-section corresponding to the cross-section shape of Fig. 19 in the rubber plug 65 is formed as shown in Fig. 22 may also be used. When viewing this rubber plug 75 from the bottom surface side as shown in Fig. 21, the bulging-out part 71 is formed in a symmetrical spindle-shape, interposing the slit 75a and having a width larger at the slit 75a and becoming smaller toward both sides. The shapes of the other portions in this rubber plug 75 are the same as the rubber plug 65. Thus, the identical notations are assigned to the identical parts and the detailed descriptions are omitted. With the liquid co-injection apparatus comprising this rubber plug 75, functions and effects similar to those of the liquid co-injection apparatus comprising the rubber plug 65 may also be obtained.

Moreover, the liquid co-injection apparatus according to the present invention is not limited to the aforementioned embodiments and may be arbitrarily modified and implemented accordingly within the scope of the invention. For example, a stopcock type apparatus having the valve body 30 is used as a liquid co-injection apparatus in each of the aforementioned Embodiments, however, a liquid co-injection apparatus according to the present invention may be constituted without the valve body, wherein a drug solution or the like may flow from an upstream branch-tube through to a downstream branch-tube, which is constantly in communication with the upstream branch-tube, and at the same time the other drug solution or the like may flow from the upper part branch-tube through to the chamber part. Moreover, a liquid co-injection apparatus in which branch-tubes are comprised of only the upper part branch-tube and the downstream branch-tube without the upstream branch-tube may also be used. In addition, the shape of the inner peripheral surface of the lid member 20, the shape of the rubber plug 25 or the like, and the shape, quantity and the likes of the compression projection part may also be arbitrarily altered.

### [Brief Description of the Drawings]

[Fig. 1] A plane view showing a liquid co-injection apparatus according to the first Embodiment of the present invention.
[Fig. 2] A front view of a liquid co-injection apparatus.
[Fig. 3] A side view of a liquid co-injection apparatus.
[Fig. 4] A cross-section view of the liquid co-injection apparatus shown in Fig. 2.
[Fig. 5] A cross-section view of the liquid co-injection apparatus shown in Fig. 3.
[Fig. 6] (a) is a plane view, (b) is a front view and (c) is a side view of a rubber plug.
[Fig. 7] A 7-7 cross-section view of Fig. 6 (b).
[Fig. 8] A 8-8 cross-section view of Fig. 6 (b).
[Fig. 9] A 9-9 cross-section view of Fig. 6 (b).
[Fig. 10] A 10-10 cross-section view of Fig. 6 (b).
[Fig. 11] A perspective view of a valve body.
[Fig. 12] A cross-section showing correlation between a main body and a valve body of a liquid co-injection apparatus.
[Fig. 13] A cross-section view of one side of a rubber plug comprised in the liquid co-injection apparatus according to the second Embodiment.
[Fig. 14] A cross-section view of the other side of a rubber plug comprised in the liquid co-injection apparatus according to the second Embodiment.
[Fig. 15] (a) is a front view and (b) is a side view of a rubber plug comprised in a liquid co-injection apparatus according to the third Embodiment.
[Fig. 16] A 16-16 cross-section view of Fig. 15(a).
[Fig. 17] (a) is a front view, (b) is a side view and (c) is a bottom view of a rubber plug comprised in a liquid co-injection apparatus according to the fourth Embodiment.
[Fig. 18] A cross-section view of one side of a rubber plug comprised in the liquid co-injection apparatus according to the fourth Embodiment.
[Fig. 19] A cross-section view of the other side of a rubber plug comprised in the liquid co-injection apparatus according to the fourth Embodiment.
[Fig. 20] A 20-20 cross-section view of Fig. 17(a).
[Fig. 21] A bottom view of a rubber plug according to a modified example.
[Fig. 22] A cross-section view of a rubber plug according to a modified example.

### [Description of Notation]

10...Co-injection apparatus main body, 11...chamber part, 12...Downstream branch-tube, 13...Connection opening, 14...Upstream branch-tube, 20...Lid member, 25,45,55,65,75...Rubber plugs, 25a, 45a, 65a, 75a...slit, 26, 46, 66...Rubber plug main bodies, 26a, 46a, 56a, 66a...slit forming parts, 26b,46b,66b...Dome-shaped parts, 29a,29b,29c, 29d, 59a, 59b, 59c, 59d...Ribs, 41, 61, 71...Bulging-out part, A...Liquid co-injection apparatus.

## Claims

1. A liquid co-injection apparatus comprising: a co-injection apparatus main body consisting of a chamber part, and plural branch-tubes including an upper part branch-tube extending from said chamber part to different directions respectively and extending at least upward; and a rubber plug attached to an inner peripheral surface of said upper part branch-tube, for shutting-off said upper part branch-tube by closing a slit passing through the inside as well as for allowing to communicate between inside said chamber part and inside said connecting tube by inserting a connecting tube through said slit,
wherein a compression projection part for further securing the obstructed condition of said slit when said connecting tube is not inserted into said slit by further forcing said rubber plug so as to be drawn and pressed onto the inner peripheral surface of said upper part branch-tube, is formed at a predetermined portion of said rubber plug.

2. A liquid co-injection apparatus as set forth in claim 1, wherein said compression projection part is comprised of a rib formed on the outer peripheral surface of a portion where said slit is formed.

3. A liquid co-injection apparatus as set forth in claim 1 wherein: said rubber plug main body portion is comprised of a slit forming part where said slit is formed and a dome-shaped part expanding from the lower end peripheral part to below the outer peripheral side of said slit forming part; and in addition, a bulging-out part protruding downward at the lower end peripheral part and the adjacent portion of said slit in the under surface of said dome-shaped part, is formed; and said compression projection part is comprised of said bulging-out part.
